# EUROPÄISCHE PATENTSCHRIFT

(11) **EP 0 791 368 B1**
(45) Veröffentlichungstag und Bekanntmachung des Hinweises auf die Patenterteilung: **21.06.2006**
(21) Anmeldenummer: 97102296.7
(22) Anmeldetag: 13.02.1997
(51) Int. Cl.: A61M 1/18, B01D 63/04, A61M 1/34

(54) **Dialysator**
Dialyser
Dialyseur

(30) Priorität: 26.02.1996 DE 19607162
(43) Veröffentlichungstag der Anmeldung: 27.08.1997
(73) Patentinhaber: Fresenius Medical Care Deutschland GmbH, 61352 Bad Homburg (DE)
(72) Erfinder: Gross, Arnold, 66629 Oberkirchen (DE); Wiesen, Gerhard, 66606 St. Wendel (DE)
(74) Vertreter: Laufhütte, Dieter

(56) Entgegenhaltungen:
- EP-A- 0 189 561
- EP-A- 0 233 848
- EP-A- 0 264 695
- EP-A- 0 692 268
- FR-A- 2 506 626
- FR-A- 2 626 180

## Beschreibung

Die Erfindung betrifft einen Dialysator, der insbesondere zum Einsatz beim Hämodiafiltrations-Verfahren bestimmt ist.

Die Hämodiafiltration ist ein Kombinationsverfahren, bei dem man eine Hämodialyse und Hämofiltration gleichzeitig durchführt. Dieses in EP 0 692 268 A1 beschriebene Verfahren verbindet die Vorteile des konvektiven Massentransport bei der Hämofiltration und den des dialytischen Diffusionsprozesses. Bei dem bekannten on-Line Hämodiafiltrationsverfahren nutzt man zur Bilanzierung aus, daß auch die Substitutionflüssigkeit (Substituat) durch die Bilanzierungskammern direkt erfaßt wird. Das bedeutet, daß mit der Bilanzierungskammer der Flüssigkeitshaushalt des Patienten unter Beachtung aller zugesetzten und entzogenen Flüssigkeiten zentral gesteuert wird. Es wird bei einem On-Line Verfahren auch kein zusätzlicher Vorratsbehälter für das Substituat benötigt, da dieses zeitgleich hergestellt wird. Bei dem bekannten Verfahren wird das Substituat durch einen eigens vorgesehenen On-Line Hämodiafiltrations-Filter steril filtriert, der zusätzlich zu dem eigentlichen Hämodiafiltrations-Filter vorhanden ist. Der zusätzlich vorgesehene On-Line Hämodiafiltrationsfilter wird nicht bei jeder Behandlung gewechselt. Daher wird zur Verringerung des Kreuzkontaminationsrisikos eine eigene Antikontaminationskammer vorgesehen. Trotz dieser Maßnahme bleibt noch ein Restrisiko für eine Kreuzkontamination.

FR 2626180 A1 beschreibt eine Hämodiafiltrationsvorrichtung mit einem Gehäuse, das zwei Module umfaßt. In einem Dialysemodul ist ein Bündel von Membrankapillaren und je ein Einlaß und ein Auslaß für die zu behandelnde Flüssigkeit und ein Auslaß für das Dialysat vorgesehen. Im Filtrationsmodul ist ein Bündel von Membrankapillaren zur Sterilfilterung von Dialysat mit einem Einlaß für das Dialysat vorgesehen. Die Membrankapillaren sind in die die offenen Enden des Gehäuses verschließenden Vergußmassen eingebettet, daß sich die Kapillaren des Membrankapillarbündels im Dialysemodul mit ihren beiden stirnseitig offenen Enden durch die Vergußmassen hindurch erstrecken und sich die Kapillaren des Membrankapillarbündels im Filtrationsmodul mit ihren ersten stirnseitig offenen Enden durch die Vergußmasse hindurch erstrecken, während sie mit den gegenüberliegenden zweiten stirnseitig offenen Enden in der Vergußmasse eingebettet sind. Das Dialysat, das über den Dialysateinlaß in die Membrankapillaren des Filtrationsmoduls eingetreten ist, durchdringt die Wände dieser Membrankapillaren und wird durch diese Wände filtriert. Durch eine Öffnung gelangt das so filtrierte Dialysat in das Dialysemodul in den Raum, der die Membrankapillaren des Dialysemoduls umgibt. Ein Teil des gereinigten Dialysats dringt durch die Wände der Membrankapillaren des Dialysemoduls in dessen Membrankapillaren ein.

FR 2506626 A beschreibt einen Dialysator mit zwei Modulen, die Membrankapillaren aufweisen, die jeweils auf beiden Stirnseiten offen sind. EP 0 233 848 A2 und EP 0 264 695 beschreiben Vorrichtungen zur Blutreinigung mit Hilfe von Membransystemen ohne Verwendung von Substituaten.

EP 0 189 561 und EP 0 692 268 A1 beschreiben die Verwendung von getrennten Einheiten für die Substituatfilterung und die Dialyse.

Aufgabe der vorliegenden Erfindung ist es, einen verbesserten Dialysator zu schaffen, bei dem das Kreuzkontaminationsrisiko möglichst ausgeschaltet wird und zusätzlich der Aufbau und die Bedienung des Hämodiafiltrationssystemes vereinfacht werden.

Diese Aufgabe wird mit einem Dialysator mit den Merkmalen des Anspruches 1 gelöst. Unteransprüche sind auf vorteilhafte Ausgestaltungen gerichtet.

Der erfindungsgemäße Dialysator vereinigt eine übliche Standard-Hämodialyseeinheit mit einer für die On-Line Hämodiafiltration notwendigen Sterilfiltrationseinheit für das Substituat. Der gesamte Dialysator ist als Wegwerfteil konzipiert, so daß eine Desinfektion des Moduls und dadurch bedingte Desinfektionsschädigungen beim Gehäuse oder der Membran oder infundierbare Rückstände vermieden werden. Insbesondere die Kreuzkontaminationsgefahr, die bei dem vorbekannten System bestand, ist beseitigt.

Ein weiterer Vorteil besteht darin, daß eine Vorabdichtheitsprüfung des früher separat vorzusehenden On-Line Filters für das Substituat entfällt. Dies geht mit einer kürzeren Vorbereitungszeit und einer dadurch bedingten besseren Verfügbarkeit einher. Gegenüber dem eingangs erläuterten bekannten System, bei dem eine wiederholte Dead-End Filtration vorgenommen wurde, ist keine Aufkonzentration von Endotoxinen mehr möglich.

Da der gesamte Dialysator als Wegwerfteil konzipiert ist, ist auch eine unkontrollierte, über die Empfehlungen hinausgehende Einsatzdauer des Dialysators nicht mehr möglich. Damit ist Fehlbedienungen vorgebeugt und es entfallen alle zeit- und zyklusbedingten kritischen Zustände. Das zur Förderung des steril zu filtrierenden Substituats benötigte Schlauchsystem kann nunmehr sehr einfach aufgebaut werden. Die im früheren System vorzusehende Antikontaminationskammer kann entfallen. Der Dialysator ist zu allen Schlauchsystemen und Maschinen kompatibel. Schließlich wird durch die Einsparung des zusätzlichen On-Line Filters eine entsprechende Aufhängung und der zugehörige Platz eingespart, so daß das Gesamtsystem einfacher und übersichtlicher aufgebaut ist.

Gemäß einer vorteilhaften Ausgestaltung der Erfindung besteht der Dialysator aus einem langgestreckten Gehäuse, das an seinen beiden Enden jeweils durch Deckel verschlossen ist. Die beiden Module in dem langgestreckten Gehäuse werden durch eine Trennwand voneinander abgeteilt. Die in den Modulen in Gehäuselängsrichtung angeordneten Membrankapillaren sind jeweils in Vergußmassen eingebettet, die die offenen Enden des langgestreckten Gehäuses verschließen. Dabei erstrecken sich die Kapillaren des Membrankapillarbündels im ersten Modul mit ihren stirnseitig offenen Enden durch die Vergußmassen hindurch. Die Kapillaren des Membrankapillarbündels erstrecken sich im zweiten Modul mit ihren ersten stirnseitig offenen Enden durch die Vergußmasse hindurch, während sie mit den gegenüberliegenden zweiten stirnseitig offenen Enden in der Vergußmasse eingebettet und durch diese verschlossen sind. Alternativ dazu können sich die ersten und zweiten Enden der Kapillaren durch die Vergußmassen hindurch erstrecken. Die zweiten Enden sind bei dieser Ausführungsform durch einen entsprechend angepaßten Deckel verschlossen. Durch diese Anordnungen kann im zweiten Modul das Substituat durch die Membrankapillaren steril filtriert werden und durch die ersten offenen Enden der Membrankapillaren mit der zu behandelnden Flüssigkeit, das heißt dem Blut, vereinigt werden.

Dabei können die im zweiten Modul angeordneten Membrankapillaren mit ihren ersten offenen Enden in einer zwischen der Vergußmasse und dem Deckel gebildeten Kammer, das heißt dem Flanschraum, münden, wobei der Deckel mit dem Einlaß für die zu behandelnde Flüssigkeit, das heißt dem Blut, verbunden ist. Hier handelt es sich um die sogenannte pre-dilution.

Andererseits können die im zweiten Modul angeordneten Membrankapillaren mit ihrem ersten offenen Ende in einer zwischen der Vergußmasse und dem Deckel gebildeten Kammer, das heißt Flanschraum, münden, wobei der Deckel mit dem Auslaß für die zu behandelnde Flüssigkeit verbunden ist. In diesem Fall erfolgt eine post-dilution.

Besonders vorteilhaft ist die Verwendung des erfindungsgemäßen Dialysators in einem On-Line Hämodiafiltrationsverfahren.

Weitere Einzelheiten und Vorteile der Erfindung werden anhand von in der Zeichnung dargestellten Ausführungsbeispielen näher erläutert. Es zeigen:
- Fig. 1:: einen Längsschnitt durch eine erste Ausführungsform eines erfindungsgemäßen Dialysators,
- Fig. 2:: einen Längsschnitt durch eine zweite Ausführungsform des erfindungsgemäßen Dialysators,
- Fig. 3:: ein Systemschema einer On-Line Hämodiafiltration, in welcher der erfindungsgemäße Dialysator eingesetzt ist.

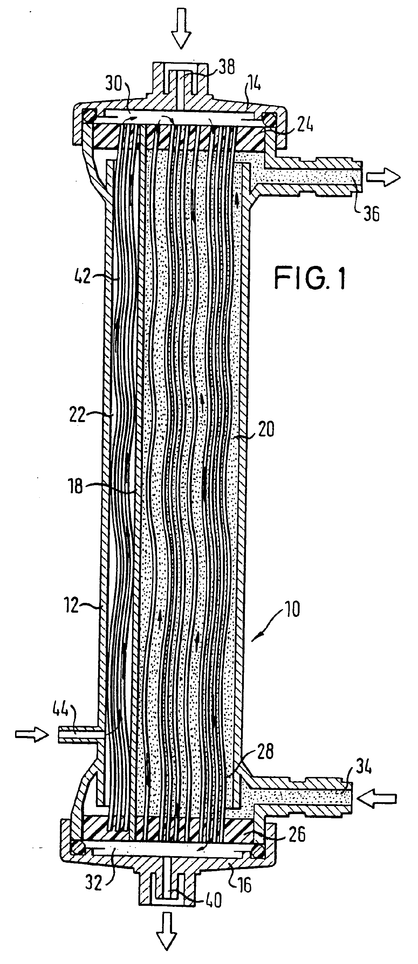

Der Dialysator 10 gemäß der in Fig. 1 dargestellten Ausführungsform umfaßt ein zylindrisches und langgestrecktes Gehäuse 12, das endseitig mit Deckeln 14 und 16 verschlossen ist. Das langgestreckte Gehäuse ist über eine Trennwand 18 in zwei Module 20 und 22 unterteilt. Die Module 20 und 22 werden jeweils einerseits durch die Trennwand und die Außenwandung des Gehäuses 12 und andererseits durch das Gehäuse 12 endseitig verschließende Vergußmassen 24 und 26 begrenzt. Das Modul 20 beinhaltet in an sich bekannter Art und Weise ein Mikrokapillarbündel mit Mikrokapillaren 28, die, wie in Fig. 1 dargestellt, längs des langgestreckten Gehäuses 12 verlaufen und in den Vergußmassen 24 und 26 derart eingebettet sind, daß ihre offenen Enden jeweils in zwischen den Vergußmassen 24 und 26 und den Deckeln 14 und 16 gebildeten Hohlräumen oder Kammern 30, 32 münden. Das Modul 20 weist einen Einlaß 34 und einen Auslaß 36 für das Dialysat in der in Fig. 1 dargestellten Art und Weise auf. Ein Einlaß 38 für das Blut ist zentrisch am Deckel 14 ausgebildet, während ein Auslaß 40 für das Blut zentrisch am Deckel 16 ausgebildet ist.

Auch das Modul 22 weist ein Kapillarbündel mit Membrankapillaren 42 auf. Diese Membrankapillaren 42 erstrecken sich ebenfalls langgestreckt zum zylindrischen Gehäuse 12. Sie sind mit einem Ende derart in die Vergußmasse 26 eingebettet, daß sie durch diese verschlossen sind. Das gegenüberliegende Ende der Membrankapillaren 42 ist in der Vergußmasse 24 eingebettet, jedoch mit den Enden zur Kammer 30 zwischen der Vergußmasse 24 und dem Dekkel 14 hin offen.

Bei dem in Fig. 1 dargestellten Dialysator tritt das zu reinigende Blut im Einlaß 38 ein und wird in der Kammer 30 mit sterilfiltriertem Substituat vereinigt. Das mit dem Substituat vermengte Blut tritt in die Membrankapillaren 28 ein und durchläuft diese bis zu deren gegenüberliegendem Ende, das in die Kammer 32 mündet. Über die Kammer 32 und den Auslaß 40 tritt das gereinigte Blut aus. Innerhalb des Moduls 20 wird das Dialysat über den entsprechenden Einlaß 34 eingeleitet und läuft im Gegenstrom zu dem zu reinigenden Blut, bis es am gegenüberliegenden Ende des Moduls 20 aus dem Auslaß 36 wieder austritt. Im Modul 22 tritt das zu behandelnde Substituat in Pfeilrichtung über einen Einlaß 44 ein. Innerhalb des Moduls 22 wird das zu reinigende Substituat über die Membrankapillaren 42 sterilfiltriert. Das sterilfiltrierte Substituat tritt am offenen Ende der Membrankapillaren, wie zuvor beschrieben, in die Kammer 30 aus und wird dort mit dem zu behandelnden Blut gemischt. Durch die in Fig. 1 gezeigten Pfeile wird jeweils die Strömungsrichtung der Flüssigkeitsströme angezeigt. Bei dem Ausführungsbeispiel gemäß Fig. 1, in welchem das Substituat mit dem einströmenden noch zu reinigenden Blut vereinigt wird, ist eine pre-dilution verwirklicht.

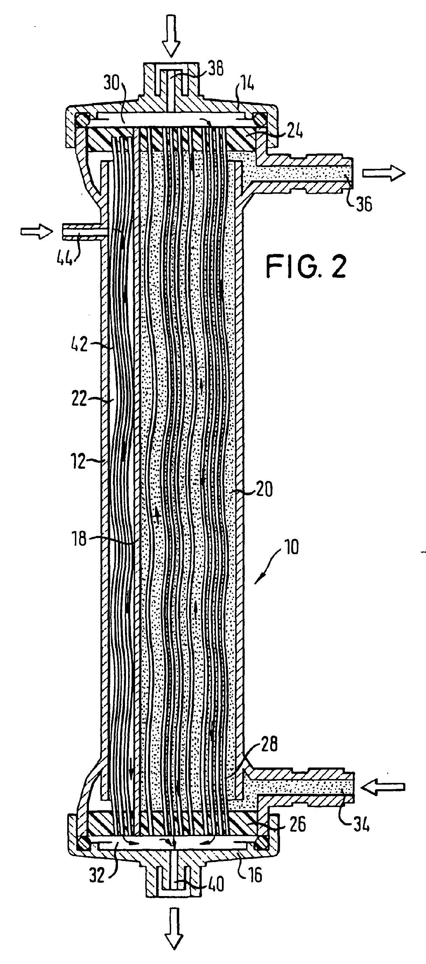

Das Ausführungsbeispiel gemäß Fig. 2 entspricht weitgehend demjenigen gemäß Fig. 1. Daher sind gleiche Teile auch mit gleichen Bezugszeichen versehen. Insbesondere der Gesamtaufbau des Gehäuses 12 und der Deckel 14 bzw. 16, sowie des Moduls 20 entspricht dem zuvor beschriebenen Ausführungsbeispiel. Das Ausführungsbeispiel unterscheidet sich aber im Aufbau des zweiten Moduls 22. Hier mündet das offene Ende der Kapillaren in die Kammer 32, die zwischen der Vergußmasse 26 und dem Deckel 16 gebildet ist. Das gegenüberliegende Ende der Membrankapillaren ist in der Vergußmasse 24 derart eingebettet, daß die Enden verschlossen sind. Weiterhin ist im Unterschied zum Ausführungsbeispiel gemäß Fig. 1 der Einlaß 44 für das zu reinigende Substituat auf der gegenüberliegenden Seite des langgestreckten Gehäuses 12 angeordnet, so daß das zu reinigende Substituat auch in dieser Ausführungsform möglichst über die gesamte Länge des Gehäuses 12 fließt (vgl. Pfeilrichtung) und über diesen Weg durch Sterilfiltration mittels der Membranwände gereinigt werden kann. Bei diesem Ausführungsbeispiel wird in der Kammer 32 das gereinigte Blut mit dem Substituat vereinigt, so daß hier eine post-dilution verwirklicht ist.

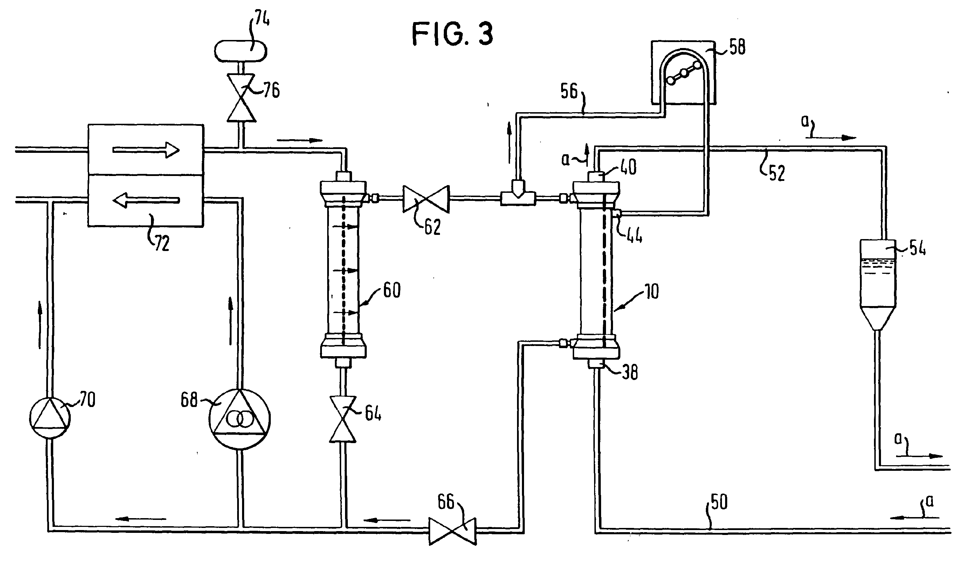

In Fig. 3 ist grundsätzlich der Aufbau eines On-Line Hämodiafiltrationssystems gezeigt. In Pfeilrichtung a wird das vom Patienten abgenommene Blut über eine Leitung 50 dem Einlaß 38 des Dialysators 10 zugeführt. Das gereinigte Blut wird in Pfeilrichtung a über den Auslaß 40 und die Leitung 52 in eine Tropfkammer 54 geleitet, von wo aus sie dem Patienten wieder zugeführt wird. Das Substituat wird über eine Leitung 56 und eine Substituatpumpe 58 in den Einlaß 44 des Dialysators 10 eingespeist. Der Teil des On-Line Hämodiafiltrationssystems gemäß Fig. 3, der die Aufbereitung des Dialysats betrifft, entspricht den bereits bekannten Systemen. So umfaßt dieser Teil ein Dialysierflüssigkeitsfilter 60, ein Dialysatorventil 62, ein Bypassventil 64, ein Ventil 66, eine Dialysatflußpumpe 68, eine UF-Pumpe 70, eine Bilanzkammer 72, ein Hydrophobfilter 74 und ein Belüftungsventil 76.

Das On-Line Hämodiafiltrationssystem gemäß der Fig. 3 ist aufgrund des Einsatzes des erfindungsgemäßen Dialysators 10 gegenüber dem vorbekannten Verfahren deutlich vereinfacht, da hier ein gesonderter Filter für das Substituat und eine diesem nachgeordnete zusätzliche Antikontaminationskammer nicht mehr notwendig sind.

## Patentansprüche

1. Dialysator (10) bestehend aus zwei in einem Gehäuse (12) verwirklichten, durch eine Trennwand (18) voneinander abgeteilten Modulen (20, 22), wobei
- das erste Modul (20) ein Bündel von Membrankapillaren (28) und je einen Einlaß (38) und einen Auslaß (40) für die zu behandelnde Flüssigkeit und einen Einlaß (34) und einen Auslaß (36) für das Dialysat aufweist,
- das zweite Modul (22) ebenfalls ein Bündel von Membrankapillaren (42) zur Sterilfiltrierung von Substituat und einen Einlaß (44) für das Substituat umfaßt,
- der Dialysator (10) eine Kammer (30; 32) aufweist, in der das gereinigte Substituat mit der zu behandelnden Flüssigkeit vereinigbar ist,
- die Membrankapillaren (28, 42) in die die offenen Enden des Gehäuses (12) verschließende Vergußmassen (24, 26) derart eingebettet sind, daß
- sich die Kapillaren (28) des Membrankapillarbündels im ersten Modul (20) mit ihren beiden stirnseitig offenen Enden durch die Vergußmassen (24, 26) hindurch erstrecken, und
- sich die Kapillaren (42) des Membrankapillarbündels im zweiten Modul (22) mit ihren ersten stirnseitig offenen Enden durch die Vergußmasse (24; 26) hindurch in die Kammer (30, 32) erstrecken, während sie mit den gegenüberliegenden zweiten stirnseitig offenen Enden in der Vergußmasse (26; 24) eingebettet und durch diese verschlossen sind, oder sich mit ihren zweiten stirnseitig offenen Enden durch die Vergußmasse hindurch erstrekken und durch einen entsprechend aufgebauten Deckel verschlossen sind.

2. Dialysator nach Anspruch 1, **dadurch gekennzeichnet, daß** das Gehäuse (12) langgestreckt ist und an seinen beiden Enden jeweils durch Deckel (14, 16) verschlossen ist.

## Claims

1. A dialyser (10) comprising two modules (20, 22) which are embodied in a housing (12) and which are separated from each other by a separating wall (18), wherein
- the first module (20) has a bundle of membrane capillaries (28) and an inlet (38) and an outlet (40) for the liquid to be treated and an inlet (34) and an outlet (36) for the dialysate respectively,
- the second module (22) also has a bundle of membrane capillaries (42) for sterile filtering of substituate and an inlet (44) for the substituate,
- the dialyser (10) has a chamber (30; 32) in which the cleaned substituate can be combined with the liquid to be treated,
- the membrane capillaries (28, 42) are embedded into the sealing compounds (24, 26) closing the open ends of the housing (12), in such a way that
- the capillaries (28) of the membrane capillary bundle in the first module (20) extend with their two open ends through the sealing compounds (24, 26), and
- the capillaries (42) of the membrane capillary bundle in the second module (22) extend with their first open ends through the sealing compound (24; 26) into the chamber (30, 32) while with the opposite second open ends they are embedded in and closed by the sealing compound (26; 24) or with their second open ends extend through the sealing compound and are closed by a suitably constructed cover.

2. A dialyser according to claim 1 **characterised in that** the housing (12) is elongate and is closed at its two ends by respective covers (14, 16).

## Revendications

1. Dialyseur (10) constitué de deux modules (20, 22) réalisés dans un boîtier (12) séparés l'un de l'autre par une paroi de séparation (18), où
- le premier module (20) présente un paquet de capillaires de membrane (28) et respectivement une entrée (38) et une sortie (40) pour le liquide à traiter et une entrée (34) et une sortie (36) pour le dialysat,
- le second module (22) présente également un paquet de capillaires de membrane (42) pour la filtration stérile du substituat et une entrée (44) pour le substituat,
- le dialyseur (10) présente une enceinte (30; 32) dans laquelle le substituat épuré peut être réuni avec le liquide à traiter,
- les capillaires de membrane (28, 42) sont noyés dans les masses de scellement (24, 26) fermant les extrémités ouvertes du boîtier (12) de telle sorte que
- les capillaires (28) du paquet de capillaires de membrane dans le premier module (20) s'étendent avec leurs deux extrémités ouvertes au côté frontal à travers les masses de scellement (24, 26), et
- les capillaires (42) du paquet de capillaires de membrane dans le second module (22) s'étendent avec leurs premières extrémités ouvertes au côté frontal à travers la masse de scellement (24, 26) dans l'enceinte (30, 32) tandis qu'avec les secondes extrémités opposées ouvertes au côté frontal ils sont noyés dans la masse de scellement (26, 24) et sont fermés par celle-ci ou bien s'étendent avec leurs secondes extrémités ouvertes au côté frontal à travers la masse de scellement et sont fermés par un couvercle réalisé d'une manière correspondante.

2. Dialyseur selon la revendication 1, **caractérisé en ce que** le boîtier (12) est oblong et est fermé à ses deux extrémités à chaque fois par un couvercle (14, 16).
